Europäisches Patentamt

European Patent Office

Office européen des brevets

(19)

(11) **EP 0 732 320 B1**

(12) **EUROPEAN PATENT SPECIFICATION**

(45) Date of publication and mention
of the grant of the patent:
**12.12.2001 Bulletin 2001/50**

(51) Int Cl.[7]: **C07C 51/48**, C07C 53/08,
C07C 53/02

(21) Application number: **95301812.4**

(22) Date of filing: **17.03.1995**

(54) **Method for recovering carboxylic acids from aqueous solutions**

Verfahren zur Rückgewinnung der Carbonsäuren aus wässrigen Lösungen

Procédé de récupération d'acides carboxyliques à partir des solutions aqueuses

(84) Designated Contracting States:
**BE DE ES FR GB IT**

(43) Date of publication of application:
**18.09.1996 Bulletin 1996/38**

(73) Proprietor: **GLITSCH, INC.**
**Dallas Texas 75212 (US)**

(72) Inventors:
 • **Gentry, Joseph Charles**
 **Houston, Texas 77079 (US)**
 • **McIntyre, John Carl**
 **Hackettstown, New Jersey (US)**
 • **Holmes, Timothy L.**
 **Dallas, Texas 75206 (US)**
 • **Gualy, Ronald G.**
 **Kingwood, Texas 77339 (US)**

(74) Representative: **Rackham, Anthony Charles**
**Lloyd Wise, Tregear & Co.,**
**Commonwealth House,**
**1-19 New Oxford Street**
**London WC1A 1LW (GB)**

(56) References cited:
 EP-A- 0 036 406          DE-A- 3 506 944
 US-A- 3 816 524          US-A- 4 143 066
 US-A- 5 175 357

 • INDUSTRIAL & ENGINEERING CHEMISTRY
 RESEARCH, vol.31, no.8, 1992, WASHINGTON
 US pages 1971 - 1981 J.W.ALTHOUSE ET AL.
 'ANALYSIS OF ORGANIC EXTRACTANT
 SYSTEMS FOR ACETIC ACID REMOVAL FOR
 CALCIUM MAGNESIUM ACETATE
 PRODUCTION.'

EP 0 732 320 B1

**Description**

This invention relates to methods for recovering carboxylic acids having from one to ten carbon atoms, and particularly formic acid, acetic acid, and mixtures of formic and acetic acids from aqueous solutions containing the same.

A number of industrially significant processes produce as a waste product dilute solutions of short chain carboxylic acids, particularly formic acid and acetic acid. Typically these solutions are in the range of one to three percent acid by weight. Heretofore it has been common to discard such solutions to the environment, but present day concerns about pollution are increasingly making it necessary-to clean such waste water solutions and/or to recover the acids that they contain as economically valuable byproducts of the processes.

Conventional recovery systems, such as distillation, or extractive distillation, tend to be objectionably energy intensive and uneconomical, as well as involving process difficulties such as the formation of azeotropes and difficult-to-break emulsions and the like.

There is thus a need for a method of treating waste water containing short chain carboxylic acids in a manner which will produce a waste water product which needs little or no after treatment before its ultimate discharge or reuse as process water in a plant, and recovered carboxylic acids of such purity and quality as to be saleable or reusable in a process plant, in an economical manner. The present invention is designed to fill that need.

EP-A-0 036 406 describes a process for extracting acetic acid, formic acid and the like from an aqueous solution of plant hydrolysis products. Trialkylphosphinic oxide is provided in a solvent so that the concentration of the oxide is kept below 80% by weight.

US-A-4 143 066 and US-A-5 175 357 disclose methods for the recovery of carboxylic acids from water by counter current extraction.

In accordance with the present invention, there is provided a method for recovering carboxylic acids from an aqueous solution containing at least one acid from the group consisting of carboxylic acids having from one to ten carbon atoms; comprising:

contacting the aqueous solution with a solvent consisting of mixed trialkylphosphine oxides in counter-current liquid-liquid extraction flow in a contacting step to thereby transfer the acids from the aqueous solution to the solvent, thereby producing a raffinate relatively low in acid content and a solvent relatively rich in acid content, the acid-rich solvent containing some water;

dehydrating the acid-rich solvent by applying heat thereto to separate water therefrom in a dehydration step thereby producing a water stream and a dehydrated rich solvent stream; and

stripping the acids from the dehydrated rich solvent stream in a stripping step without requiring an entraining agent by applying heat thereto to produce a recycle solvent consisting of mixed trialkylphosphine oxides for recycle to the liquid-liquid extraction flow and an acid stream containing the acids.

Typical concentrations of aqueous solutions amenable to treatment by the method of the invention are one to three percent by weight acids.

The substantially pure water raffinate can be passed to further treatment, disposal or reuse.

The water stream resulting from the dehydration of the acid-rich solvent is preferably recycled to the incoming aqueous solution.

The removed acids are then split into components which are essentially single species of desired purity by distillation, and are thus marketable products or products usable within a plant for other processes.

The preferred solvent extractant for use in the invention is a mixture of four trialkylphosphine oxides, manufactured by Cytech Industries under the trademark CYANEX 923.

The preferred solvent extractant is a mixture of four trialkylphosphine oxides as follows:

$$R_3P(O) \qquad R_2R'P(O) \qquad RR'_2P(O) \qquad R'_3P(O)$$

$$\text{Where } R = [CH_3(CH_2)_7] \text{ - normal octyl}$$

$$R' = [CH_3(CH_2)_5] \text{ - normal hexyl}$$

$$\text{Average Molecular Weight} = 348 \text{ (approximately).}$$

Typical properties of this solvent are reported as follows:

| Trialkylphosphine oxides | 93% by weight |
| Appearance | Colorless mobile liquid |
| Specific Gravity | 0.88 at 23°C |
| Freezing Point | -5 to 0°C |
| Viscosity | 40.0 centipoise at 25°C |
| | 13.7 centipoise at 30°C |
| Flashpoint (Closed Cup Setaflash) | 182°C |
| Autoignition Temperature | 218°C |
| Vapor Pressure | 0.09 mm Hg at 31°C |
| Boiling Point | 310°C at 50 mm Hg (about 6.67 x $10^3$ Pa) |
| Solubility in Water | 10 mg/l |
| Solubility of Water in CYANEX 923 extractant | 8 w/o |

The solvent used according to the invention will consist of mixed trialkylphosphine oxides, although incidental ingredients and impurities may be present if they do not materially effect the action of the solvent as the above example of CYANEX 923 demonstrates.

Preferably, the recycle solvent is recycled to the contacting step, and the water stream from dehydration of the rich solvent is recycled to the incoming aqueous solution.

The volume ratio of solvent consisting of mixed trialkylphosphine oxides to aqueous solution during the contacting is from one part solvent to two parts aqueous solution to two parts solvent to one part aqueous solution. The initial concentration of acids in the aqueous solution is preferably from one-half percent (0.5%) by weight to fifteen percent (15%) by weight, although it may be from one percent (1%) by weight to ten percent (10%) by weight. It is also preferred that the recycle solvent have an acid content less than 0.5% by weight.

The contacting of the aqueous solution with the solvent is desirably performed at a temperature between 35°C and 90°C, and more preferably at a temperature between 50°C and 80°C. Further, the dehydrating step is preferably performed at a pressure of 200 millimeters of mercury absolute (approx $2 \cdot 67 \times 10^4$ Pa). It is preferred that the stripping be performed at a temperature of from 250°C to 300°C at the hottest region of the stripping step, and it is also preferred that the stripping be performed at a pressure of from the 15 to 50 millimeters of mercury absolute. The pressure at which stripping is performed is desirably sufficient to avoid freezing of any acid, particularly acetic acid, during the stripping or downstream thereof.

In order to maximize energy efficiency, heat in the recycle solvent consisting of mixed trialkylphosphine oxides from the stripping step may be transferred at least in part to the rich solvent in the dehydration step. Also, heat in the recycle solvent consisting of mixed trialkylphosphine oxides from the stripping step may be transferred at least in part to the acid stream being split into constituent acids.

Fresh solvent consisting of mixed trialkylphosphine oxides for use in the process may be purified prior to being contacted with the aqueous solution, for example by water washing or by distillation.

Although several modes of operation are possible, it is preferred that the contacting be effected by dispersing the solvent consisting of mixed trialkylphosphine oxides as a dispersed phase in the aqueous solution which is then the continuous phase.

Vapours vented from the dehydration and/or the stripping steps may be scrubbed with scrubbing solvent consisting of mixed trialkylphosphine oxides employed in the contacting step, and acids thereby dissolved in the scrubbing solvent may thereafter be recovered therefrom.

Any solvent consisting of mixed trialkylphosphine oxides which becomes entrained in the raffinate from the contacting step may be coalesced out of the raffinate, thus increasing raffinate purity and recovering solvent for reuse.

Undesirable water in the final acid products may be reduced or eliminated by providing a side stream which is drawn from the distillation splitting step at a region where the water concentration therein is greatest, or at a region where an azeotrope is formed between water and formic acid in the course of being distilled.

Impurities in the incoming aqueous solution which may interfere with the process may be eliminated by a pretreatment in which rich solvent from the contacting step is mixed with incoming aqueous solution prior to its delivery to the contacting step and then the rich solvent and aqueous solution are separated from each other by coalescing the rich solvent from the aqueous solution, with the separated aqueous solution then being delivered to the contacting step and the separated rich solvent being delivered to the dehydration step.

Impurities tending to accumulate in the recycle solvent may be removed therefrom as it is in the course of being recycled. These impurities may be removed by vacuum distillation of at least a portion of the solvent, or by activated carbon filtration of at least a portion of the solvent, or by contacting at least a portion of the solvent with an ion exchange agent in the course of its being recycled. Also, the impurities may be removed by neutralization with a basic additive of at least a portion of the solvent for recycle in the course of its being recycled. The impurities may also be controlled by adjusting the relative flow rates of aqueous solution and recycle solvent so that the equilibrium concentrations of impurities in the solvent and in the raffinate are each at acceptable levels.

The invention will now be illustrated with reference to the accompanying drawings, in which :

FIG. 1 is a diagrammatic flowsheet for a plant for practicing the method of the invention, the plant being fitted particularly for recovering and separating formic acid and acetic acid from a dilute aqueous solution; and
FIG. 2 is a diagrammatic flowsheet for a plant for practicing the method of the invention to recover and separate a mixture of short chain carboxylic acids, and showing certain method features which may also be employed in the plant and method of FIG. 1.

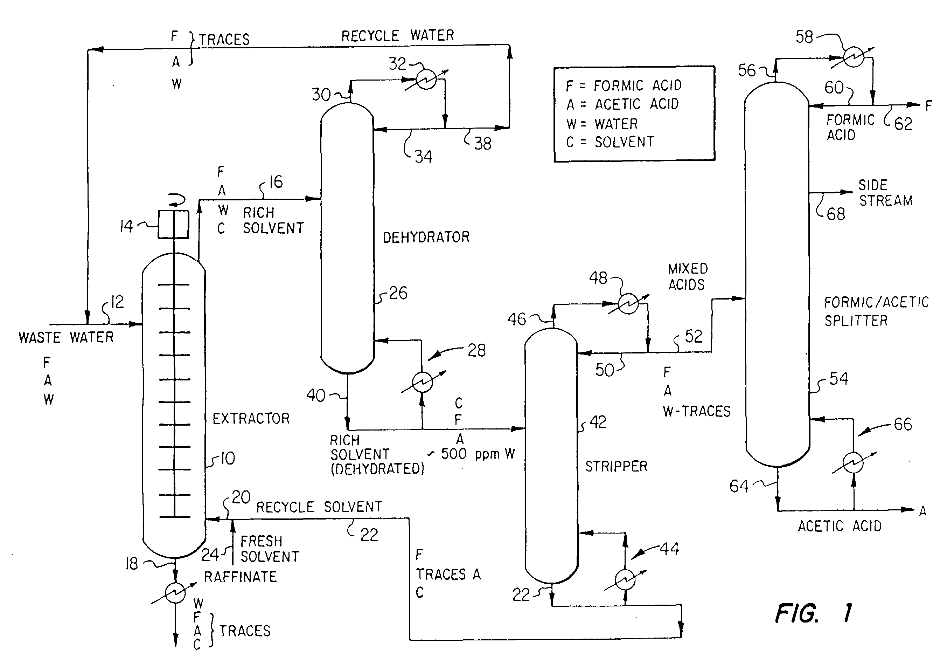

Attention is first directed to FIG. 1 which shows in simplified flow diagram form a plant arranged for the practice of the method of the invention. Aqueous solution or waste water containing the acids sought to be recovered is delivered to extractor 10 through input line 12. The extractor is shown as being of the rotating plate type, with a motor 14 provided to rotate the plates. Various other kinds of liquid-liquid extractor devices may be employed. The top stream out of the extractor 10 is a rich solvent stream that leaves the extractor through line 16, while the bottom stream is a raffinate leaving through line 18. Fresh solvent and recycle solvent are introduced into the extractor 10 near the bottom through line 20, the recycle solvent being delivered to line 20 through line 22 and fresh solvent, when needed, being delivered. through line 24. The volume ratio of solvent and aqueous solution fed to the extractor is preferably between about one-to-two and two-to-one. The initial concentration of acids in the aqueous solution feed is from 0.5% to 15%, and preferably from 1% to 6% by weight. The operational temperature in the contacting step is between 35°C and 90°C, and preferably between 50°C and 80°C. The phase separation line is preferably at the top region of the extractor, and the dispersed phase is the solvent while the continuous phase is the aqueous solution.

The rich acid containing solvent is delivered through line 16 to dehydrator 26. Heat is input to the dehydrator through a reboiler system 28 and water exits the dehydrator at the top through line 30, which is provided with a condenser 32 and reflux line 34 if desired. The water stream is delivered through line 38 in a recycle circuit back to input line 12. Alternately, the water stream may be delivered out of the plant for use or disposition elsewhere or combined with the raffinate stream. The pressure in the dehydrator is preferably about 200 millimeters of mercury absolute (approx $2 \cdot 67 \times 10^4$ Pa).

Rich solvent leaves the dehydrator through line 40 which delivers it to stripper 42. In stripper 42, the acids are separated from the solvent and through application of heat through reboiler system 44, and the mixed acids exit the stripper at the top through line 46, which is provided with a condenser 48 and a recycle line 50. The mixed acids are delivered through line 52 to splitter 54. The stripped solvent leaves the bottom of stripper 42 through line 22 and is recycled to the extractor 10. The acid content of the stripped solvent for recycle is less than about 0.5% by weight. At the hottest region of the stripper, the operating temperature is from 250°C to 300°C, and the pressure in the stripper is from 15 to 50 millimeters of mercury absolute (approx. $2 \cdot 00 \times 10^3$ to $6 \cdot 67 \times 10^3$ Pa). In any event, the stripping pressure should be such as to avoid freezing of any acids there or downstream. If desired, part of the heat in the stripped solvent may be heat exchanged with rich solvent at the dehydrator reboiler system 28, and also, if desired, part of the heat in the stripped solvent may be heat exchanged with the bottom acid stream in reboiler system 66 discussed below.

The mixed acids delivered to splitter 54 through line 52 are separated in a distillation process, with formic acid exiting the splitter through line 56 and being condensed in condenser 58 and refluxed in part through line 60 with the balance being delivered out of the plant through product line 62. The acetic acid is delivered out of the bottom of the unit through line 64, and a reboiler system 66 is provided to deliver heat energy into the distillation tower or splitter 54. In accordance with the invention, a side stream 68 may be taken off the splitter at a point where the concentration of any water contaminant is at its highest; this point most usually being at a point where an azeotrope is formed between water and the lighter acid, that is, the formic acid.

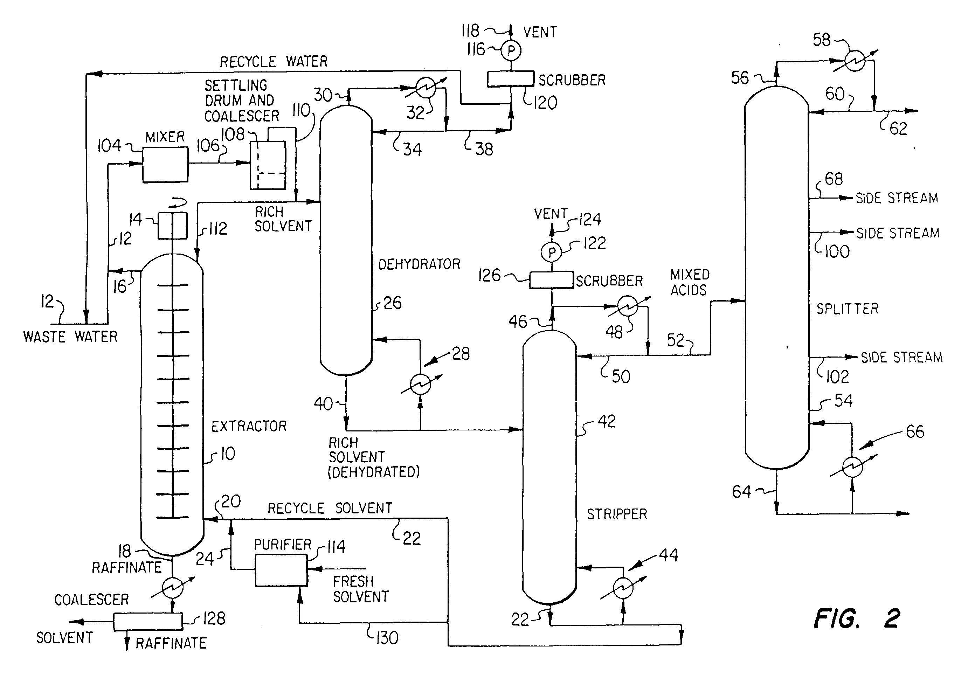

Attention is now directed to FIG. 2 which is a diagrammatic flow sheet for a plant for practicing other embodiments of the invention. In FIG. 2, pieces of equipment and lines which are identical to those in FIG. 1 are given the same reference characters, and the description of those parts given in connection with FIG. 1 applies to the plant shown in FIG. 2 also.

The plant shown in FIG. 2 differs from that of FIG. 1 in that it is designed to process an aqueous solution containing more than formic acid and acetic acid from the group of carboxylic acids having from one to five carbon atoms. Thus the splitter or distillation column 54 in FIG. 2 is provided with additional side stream take-offs 100 and 102 for recovering additional acids beyond those which the distillation operation will tend to separate to the top and bottom of that tower. Those skilled in the art will appreciate that the separation of the recovered mixed acids may be effected stage-wise in a series of towers rather than in a single tower, if desired.

It has been discovered that impurities in the waste water or input aqueous solution entering the system through line 12 may cause operational difficulties through inducing emulsification in various streams in the plant, and as well as other operating inefficiencies. In accordance with the invention, these impurities may be eliminated by an additional process step in which rich solvent leaving extractor 10 through line 16 is joined with fresh incoming aqueous solution in line 12 and these materials are passed to mixer 104. After being thoroughly mixed, the rich solvent and the incoming aqueous solution pass through line 106 to a coalescer 108 where the species separate, and rich solvent then passes through line 110 to the dehydrator 26, while acid containing water is fed through line 112 into extractor 10. In this manner, the impurities in the incoming aqueous solution are made more readily coalescible.

It has also been found that unexpected impurities may be in the fresh solvent which is charged to the plant at startup, or which is added to the plant in the course of operations. As appears in FIG. 2, a purifier 114 may be placed in the fresh solvent input line 24 to remove these impurities. The purifier 114 may be a distillation tower, an activated carbon bed, an ion exchange system, a water wash system, or other separating equipment. Since the problem of impurities in the fresh solvent is most acute at startup, piping may be provided to utilize equipment of the plant during startup to effect the removal of the impurities in the fresh solvent. For example, stripper 42 or dehydrator 26 may be temporarily so employed during startup. Additionally, the impurities may be removed in a purifier which is a separate stand-alone plant, if desired.

As has been explained above, it is preferred that dehydrator 26 and stripper 42 be operated at vacuum. The vacuum inducing equipment is shown in FIG. 2, although it should be understood that similar equipment would be present in a plant arranged as in FIG. 1. This equipment includes pump 116 and vent 118 in line 38 out of the top of the dehydrator 26. The pump may be a mechanical pump or other vacuum inducing equipment such as a steam ejector. Since the stream through vent 118 may contain some acids and other material which would be viewed as pollutants, a scrubber 120 may be provided. Scrubber 120 may use as the scrubbing fluid the same kind of solvent as is employed in the plant as the main extractant, and the solvent may be passed through scrubber 120 as a side loop in the main solvent circuit of the plant.

Similarly for tower 42, a vacuum pump 122 and vent 124 are provided. Also, a scrubber 126 is provided for removing this acid and other pollutants from the stream leaving the plant through vent 124.

As has been noted above, impurities may tend to accumulate in the solvent as it is cycled through the process. These impurities may be conveniently removed from the recycle solvent passing through line 22, by drawing off at least a portion of the recycle solvent through line 130 and passing it through purifier 114, or through a separate purifier, where the impurities may be removed by vacuum distillation, activated carbon filtration, ion exchange, or neutralization with a basic additive, or otherwise.

Under some operating conditions, the raffinate leaving the extractor 10 through line 18 may have some solvent physically entrained in it. This solvent can be removed and recovered, for example in a coalescer 128. The recovered solvent may be returned to the main solvent circuit of the plant, and the raffinate, now freed of entrained solvent may be discarded, further processed, or reused in the plant.

Various features of the plant illustrated in FIG. 2 which is designed for processing multiple acids in the incoming aqueous solution stream may be incorporated into the plant shown in FIG. 1, which is designed for processing formic acid and acetic acid only, and the converse is the case as will be appreciated by those skilled in the art.

## Claims

1.   A method for recovering carboxylic acids from an aqueous solution containing at least one acid from the group consisting of carboxylic acids having from one to ten carbon atoms; comprising:

   contacting the aqueous solution with a solvent consisting of mixed trialkylphosphine oxides in counter-current liquid-liquid extraction flow in a contacting step to thereby transfer the acids from the aqueous solution to the solvent, thereby producing a raffinate relatively low in acid content and a solvent relatively rich in acid content, the acid-rich solvent containing some water;
   dehydrating the acid-rich solvent by applying heat thereto to separate water therefrom in a dehydration step thereby producing a water stream and a dehydrated rich solvent stream; and
   stripping the acids from the dehydrated rich solvent stream in a stripping step without requiring an entraining agent by applying heat thereto to produce a recycle solvent consisting of mixed trialkylphosphine oxides for recycle to the liquid-liquid extraction flow and an acid stream containing the acids.

2.   A method as claimed in Claim 1 for recovering acetic acid and formic acid and further comprising splitting the acid stream into acetic acid and formic acid in a splitting step by distillation.

3. A method in accordance with Claim 1 or Claim 2 in which the recycle solvent is recycled to the contacting step.

4. A method in accordance with any preceding claim in which the water stream from dehydration of the rich solvent is recycled to the aqueous solution.

5. A method in accordance with any preceding claim in which the volume ratio of solvent consisting of mixed trialkylphosphine oxides to aqueous solution during contacting is from one part solvent to two parts aqueous solution to two parts solvent to one part aqueous solution.

6. A method in accordance with any preceding claim in which the initial concentration of acids in the aqueous solution is from one-half percent (0.5%) by weight to fifteen percent (15%) by weight.

7. A method in accordance with Claim 6 in which the initial concentration of acids in the said aqueous solution is from one percent (1%) by weight to six percent (6%) by weight.

8. A method in accordance with any preceding claim in which the recycle solvent has an acid content less than 0.5% by weight.

9. A method in accordance with any preceding claim in which the contacting of the aqueous solution with the solvent is performed at a temperature between 50°C and 80°C.

10. A method in accordance with Claim 9 in which the contacting of aqueous solution with solvent is performed at a temperature between 35°C and 90°C.

11. A method in accordance with any preceding claim in which the dehydrating step is performed at a pressure of 200 millimetres of mercury absolute (approx 27 x 10³ Pa).

**Patentansprüche**

1. Verfahren zur Rückgewinnung von Carbonsäuren aus einer wäßrigen Lösung, die zumindest eine Säure aus der Gruppe enthält, die aus Carbonsäuren mit einem bis zehn Kohlenstoffatomen besteht, beinhaltend In-Kontakt-Bringen der wäßrigen Lösung mit einem Lösungsmittel, das aus gemischten Trialkylphosphinoxiden in einem Flüssig-Flüssig-Extraktionsgegenstromverfahren in einem Kontaktschritt, um so die Säuren von der wäßrigen Lösung in das Lösungsmittel zu überführen, wobei ein Raffinat mit einem relativ geringen Säuregehalt und ein Lösungsmittel mit einem relativ hohen Säuregehalt erzeugt wird, wobei das säurereiche Lösungsmittel etwas Wasser enthält, Dehydrieren des säurereichen Lösungsmittels, indem Wärme zugeführt wird, um Wasser in einem Dehydrierungsschritt von diesem abzutrennen, wobei ein Wasserstrom und ein wasserfreier reicher Lösungsmittelstrom erzeugt werden, und Abtrennen der Säuren aus dem wasserfreien reichen Lösungsmittelstrom in einem Trennschritt, ohne daß ein Schleppmittel notwendig ist, indem Wärme zugeführt wird, um ein rückgewonnenes Lösungsmittel, das aus gemischten Trialkylphosphinoxiden besteht, zur Rückführung in den Flüssig-Flüssig-Extraktionsstrom, und einen Säurestrom, der die Säuren enthält, zu erzeugen.

2. Verfahren nach Anspruch 1 zur Rückgewinnung von Essigsäure und Ameisensäure, welches in einem Trennschritt eine Trennung des Säurestroms in Essigsäure und Ameisensäure durch Destillation beinhaltet.

3. Verfahren nach Anspruch 1 oder Anspruch 2, in dem das rückgewonnene Lösungsmittel in den Kontaktschritt zurückgeführt wird.

4. Verfahren nach einem der vorhergehenden Ansprüche, in dem der Wasserstrom aus dem Dehydrierungsschritt des reichen Lösungsmittels in die wäßrige Lösung zurückgeführt wird.

5. Verfahren nach einem der vorhergehenden Ansprüche, in dem das Volumenverhältnis von Lösungsmittel, das aus gemischten Trialkylphosphinoxiden besteht, zu wäßriger Lösung während des Kontaktvorgangs im Bereich von einem Teil Lösungsmittel und zwei Teilen wäßrigem Lösungsmittel bis zwei Teilen Lösungsmittel und einem Teil wäßriger Lösung liegt.

6. Verfahren nach einem der vorangehenden Ansprüche, in dem die Konzentration der Säuren zu Beginn in der wäßrigen Lösung im Bereich von einem halben Gewichtsprozent (0,5 %) bis 15 Gewichtsprozent (15 %) liegt.

7. Verfahren nach Anspruch 6, in dem die Konzentration an Säuren in der wäßrigen Lösung im Bereich von einem Gewichtsprozent (1%) bis sechs Gewichtsprozent (6 %) liegt.

8. Verfahren nach einem der vorhergehenden Ansprüche, in dem das rückgeführte Lösungsmittel einen Säuregehalt von weniger als 0,5 Gew.-% hat.

9. Verfahren nach einem der vorhergehenden Ansprüche, in dem das In-Kontakt-Bringen von wäßriger Lösung und Lösungsmittel bei einer Temperatur zwischen 50°C und 80°C erfolgt.

10. Verfahren nach Anspruch 9, in dem das In-Kontakt-Bringen von wäßriger Lösung und Lösungsmittel bei einer Temperatur zwischen 35°C und 90°C erfolgt.

11. Verfahren nach einem der vorhergehenden Ansprüche, in dem der Dehydrierungsschritt bei einem absoluten Druck von 200 Millimeter Quecksilbersäule (etwa 27 x 10³ Pa) durchgeführt wird.

**Revendications**

1. Procédé pour récupérer des acides carboxyliques à partir d'une solution aqueuse contenant au moins un acide choisi dans l'ensemble constitué des acides carboxyliques ayant de 1 à 10 atomes de carbone, comprenant :

   la mise en contact de la solution aqueuse avec un solvant consistant en un mélange d'oxydes de trialkylphosphine, dans un écoulement d'extraction liquide-liquide à contre-courant dans une étape de mise en contact pour transférer ainsi les acides de la solution aqueuse au solvant, de façon à produire un raffinat ayant une teneur relativement faible en acides et un solvant relativement riche en acides, le solvant riche en acides contenant un peu d'eau ;
   la déshydratation du solvant riche en acides par application de chaleur à ce solvant pour en séparer l'eau dans une étape de déshydratation de façon à produire un courant d'eau et un courant de solvant riche déshydraté ; et
   la séparation des acides du courant de solvant riche déshydraté dans une étape de séparation n'exigeant pas d'agent d'entraînement, par application de chaleur pour produire un solvant de recyclage consistant en un mélange d'oxydes de trialkylphosphine pour recyclage vers l'écoulement d'extraction liquide-liquide, et un courant d'acide contenant les acides.

2. Procédé selon la revendication 1, pour récupérer de l'acide acétique et de l'acide formique, et comprenant en outre la dissociation du courant d'acide en acide acétique et en acide formique dans une étape de dissociation par distillation.

3. Procédé selon la revendication 1 ou 2, dans lequel le solvant de recyclage est recyclé vers l'étape de mise en contact.

4. Procédé selon une revendication précédente quelconque, dans lequel le courant d'eau provenant de la déshydratation du solvant riche est recyclé vers la solution aqueuse.

5. Procédé selon une revendication précédente quelconque, dans lequel le rapport en volume du solvant consistant en un mélange d'oxydes de trialkylphosphine à la solution aqueuse pendant la mise en contact est d'une partie de solvant pour deux parties de solution aqueuse à deux parties de solvant pour une partie de solution aqueuse.

6. Procédé selon une revendication précédente quelconque, dans lequel la concentration initiale des acides dans la solution aqueuse est de un demi pour cent (0,5 %) en poids à quinze pour cent (15 %) en poids.

7. Procédé selon la revendication 6, dans lequel la concentration initiale des acides dans ladite solution aqueuse est de un pour cent (1 %) en poids à six pour cent (6 %) en poids.

8. Procédé selon une revendication précédente quelconque, dans lequel le solvant de recyclage a une teneur en acides inférieure à 0,5 % en poids.

9. Procédé selon une revendication précédente quelconque, dans lequel la mise en contact de la solution aqueuse avec le solvant est réalisée à une température comprise entre 50 et 80°C.

10. Procédé selon la revendication 9, dans lequel la mise en contact de la solution aqueuse avec le solvant est réalisée à une température comprise entre 35 et 90°C.

11. Procédé selon une revendication précédente quelconque, dans lequel l'étape de déshydratation est réalisée sous une pression absolue de 200 mm de mercure (environ 27 x 10$^3$ Pa).

F = FORMIC ACID
A = ACETIC ACID
W = WATER
C = SOLVENT

FIG. 1

FIG. 2